Europäisches Patentamt

European Patent Office

Office européen des brevets

(19)

(11) **EP 0 857 481 B1**

(12) **FASCICULE DE BREVET EUROPEEN**

(45) Date de publication et mention
de la délivrance du brevet:
**07.02.2001 Bulletin 2001/06**

(51) Int Cl.⁷: **A61K 7/48**, A61K 7/06

(21) Numéro de dépôt: **97403155.1**

(22) Date de dépôt: **24.12.1997**

(54) **Utilisation d'un organopolysiloxane pour la fixation et/ou la libération prolongée de parfum**

Verwendung eines Organopolysiloxans zur Fixierung und/oder Verlängerung der Freisetzung von Parfüm

Use of an organopolysiloxane for the fixing and/or the prolonged release of perfume

(84) Etats contractants désignés:
**DE ES FR GB IT**

(30) Priorité: **31.01.1997 FR 9701105**

(43) Date de publication de la demande:
**12.08.1998 Bulletin 1998/33**

(73) Titulaire: **L'OREAL**
**75008 Paris (FR)**

(72) Inventeurs:
 • **Auguste, Frédéric**
  **94550 Chevilly Larue (FR)**

 • **Bara, Isabelle**
 **75013 Paris (FR)**

(74) Mandataire: **Tezier Herman, Béatrice**
**L' OREAL,**
**D.P.I.,**
**6, rue Bertrand Sincholle**
**92585 Clichy Cédex (FR)**

(56) Documents cités:
**EP-A- 0 118 625     EP-A- 0 295 886**
**EP-A- 0 790 055     WO-A-90/10436**
**US-A- 4 374 236     US-A- 5 266 322**

**Description**

La présente invention se rapporte à l'utilisation d'au moins un organopolysiloxane élastomérique solide partiellement réticulé associé à une phase grasse, dans une composition pour la fixation et/ou la libération prolongée d'un parfum. L'invention se rapporte aussi à un procédé de traitement de la peau, aussi bien du visage que du corps, et/ou des cheveux par application de la composition obtenue. Cette dernière peut être aussi bien une composition cosmétique qu'une composition dermatologique.

On sait qu'un parfum est l'association de différentes substances odorantes qui s'évaporent à des périodes différentes. Chaque parfum présente ce que l'on appelle une « note de tête » qui est l'odeur diffusant en premier lors de l'application du parfum ou lors de l'ouverture du récipient le contenant, une « note de coeur ou corps » qui correspond au parfum complet (émission pendant quelques heures après la « note de tête ») et une « note de fond » qui est l'odeur la plus persistante (émission pendant plusieurs heures après la « note de coeur »).

L'être humain a de tout temps cherché à se parfumer et à parfumer les objets qui l'entourent ou les lieux dans lesquels il se trouve, et ceci, aussi bien pour masquer des odeurs fortes et/ou désagréables que pour donner une bonne odeur.

Il est courant d'incorporer du parfum dans un certain nombre de produits ou compositions, en particulier cosmétiques et dermatologiques. Or, selon la nature de ces produits ou compositions, il n'est pas toujours aisé d'incorporer tel ou tel parfum et/ou de conserver les effets olfactifs recherchés. Pour remédier à ces inconvénients, plusieurs solutions ont déjà été envisagées.

Ainsi, il est connu par le document JP-A-01101399 un savon solide, contenant un parfum qui est libéré progressivement au cours des différentes utilisations du savon. Ce savon parfumé est obtenu en incorporant d'abord le parfum à une argile minérale composite organique puis en mélangeant l'argile parfumée à la composition de savon. Malheureusement, l'utilisation d'une argile pour maintenir le parfum dans ce savon favorise la dégradation du parfum au contact de la chaleur ou d'agent alcalin présent dans la composition.

Par ailleurs, il est connu d'utiliser les cyclodextrines, molécules cycliques, pour complexer les parfums et permettre ainsi leur libération contrôlée. De telles compositions sont décrites par exemple dans les documents EP-A-13688 et US-A-5238915. Malheureusement, les procédés d'inclusion des parfums dans les cyclodextrines sont complexes. En outre, les cyclodextrines sont des produits au toucher peu cosmétique.

De plus, le document WO-A-90/10436 décrit un agent parfumant contenant une huile essentielle encapsulée dans une membrane et obtenu à partir d'un mélange d'huile essentielle et d'organosiloxane non réticulé, et le document US-A-4,374,236 décrit des diffuseurs de parfums solides réalisés à partir d'un mélange d'élastomère non réticulé et de parfums.

Aussi, il subsiste le besoin d'une composition parfumée, en particulier cosmétique et/ou dermatologique ne présentant pas les inconvénients ci-dessus et notamment qui peut être préparée facilement, permettant d'augmenter la rémanence du parfum sans que ce dernier se dégrade, en particulier au contact des autres constituants de la composition.

La demanderesse a découvert de façon surprenante que l'introduction d'un organopolysiloxane élastomérique solide partiellement réticulé dans une composition parfumée permettait au parfum de ne pas se dégrader et de persister plusieurs heures sur le sujet ou l'objet à parfumer.

Aussi, l'invention porte sur l'utilisation d'au moins un organopolysiloxane élastomérique solide partiellement réticulé associé à une phase grasse dans une composition contenant, dans un milieu physiologiquement acceptable, un parfum pour la fixation et/ou la libération prolongée de ce parfum, l'organo-polysiloxane élastomérique étant susceptible d'être obtenu par réaction d'addition et de réticulation, en présence d'un catalyseur, d'au moins :

(a) un organopolysiloxane ayant au moins deux groupes alcényle inférieurs par molécule ; et
(b) un organopolysiloxane ayant au moins deux atomes d'hydrogène liés à un atome de silicium par molécule.

Par « élastomérique » on entend un matériau souple, déformable ayant des propriétés viscoélastiques et notamment la consistance d'une éponge ou d'une sphère souple.

Certes, il est connu par le document EP-A-118625 d'utiliser l'hexaméthylcyclotrisiloxane qui est une huile siliconée volatile, comme support de matières odorantes pour des désodorisants d'ambiance. Toutefois, dans ce document, l'utilisation de l'hexaméthylcyclotrisiloxane a pour but de favoriser le flottement du parfum dans l'atmosphère du fait de la volatilité de cette huile et non de fixer le parfum dans la composition le contenant.

En revanche, l'organopolysiloxane de l'invention n'est pas un composé volatil et il permet au contraire une libération du parfum, prolongée dans le temps lors de son application.

Les organopolysiloxanes élastomériques conformes à l'invention sont partiellement ou totalement réticulés. Inclus dans une phase grasse, ils se transforment, selon le taux de phase grasse utilisé, d'un produit d'aspect spongieux lorsqu'ils sont utilisés en présence de faibles teneurs en phase grasse en un gel homogène, en présence de quantités de phase grasse plus élevées. La gélification de la phase grasse par ces élastomères peut être totale ou partielle.

Les élastomères de l'invention peuvent être véhiculés sous forme de gel constitué d'un organopolysiloxane élastomère, incluant au moins une huile hydrocarbonée et/ou une huile de silicone. Aussi, la phase grasse associée à l'organopolysiloxane élastomérique peut être constituée de cette ou ces huiles.

Les organopolysiloxanes élastomériques selon l'invention peuvent être choisis parmi les polymères réticulés décrits dans la demande EP-A-0295886. Selon cette demande, ils sont obtenus par réaction d'addition et de réticulation, en présence d'un catalyseur du type platine, d'au moins :

- (a) un organopolysiloxane ayant au moins deux groupes alcényle inférieurs par molécule, ces groupes alcényle comportant deux à six atomes de carbone ; et
- (b) un organopolysiloxane ayant au moins deux atomes d'hydrogène liés à un atome de silicium par molécule.

Les organopolysiloxanes élastomériques selon l'invention peuvent aussi être choisis parmi ceux décrits dans le brevet US-A-5.266.321. Selon ce brevet, ils sont choisis notamment parmi :

- i) les organopolysiloxanes comprenant des motifs $R_2SiO$ et $RSiO_{1,5}$ et éventuellement des motifs $R_3SiO_{0,5}$ et/ou $SiO_2$, dans lesquels les radicaux R, indépendamment les uns des autres, représentent un hydrogène, un alkyle tel que méthyle, éthyle ou propyle, un aryle tel que phényle ou tolyle, un groupe aliphatique insaturé tel que vinyle, le rapport en poids des motifs $R_2SiO$ sur les motifs $RSiO_{1,5}$ allant de 1/1 à 30/1 ;
- ii) les organopolysiloxanes insolubles et gonflables dans l'huile de silicone, obtenus par addition d'un organohydrogénopolysiloxane (1) et d'un organopolysiloxane (2) ayant des groupes aliphatiques insaturés de telle sorte que la quantité d'hydrogène ou de groupes aliphatiques insaturés dans respectivement (1) et (2) soit comprise entre 1 et 20 mole % lorsque l'organopolysiloxane est non cyclique et entre 1 et 50 mole % lorsque l'organopolysiloxane est cyclique.

Les organopolysiloxanes de la composition de l'invention sont par exemple ceux commercialisés sous les noms KSG6 de Shin-Etsu, Trefil E-505C ou Trefil E-506C de Dow-Corning, Gransil (SR-CYC, SR DMF10, SR-DC556) de Grant Industries, ou ceux commercialisés sous forme de gels déjà constitués : KSG15, KSG17, KSG16, KSG18, KSG26A, KSG26B, de Shin-Etsu, Gransil SR 5CYC gel, Gransil SR DMF 10 gel, Gransil SR DC556 gel de Grant Industries, 1229-02-167 et 1229-02-168 de General Electric. On peut aussi utiliser un mélange de ces produits commerciaux.

Les organopolysiloxanes élastomériques utilisés selon l'invention ne sont pas desséchants pour la peau et apportent de bonnes propriétés cosmétiques. Ils conduisent à des compositions confortables à l'application, douces et non collantes au toucher. Cette douceur est due notamment à la texture des organopolysiloxanes. En outre, ils permettent de fixer le parfum et d'obtenir une très bonne rémanence de la composition obtenue, notamment lorsque cette composition se présente sous forme d'un gel.

De façon préférentielle, la phase grasse associée à l'organopolysiloxane élastomérique comprend une ou plusieurs huiles hydrocarbonées et/ou de silicone et/ou fluorées. Ces huiles peuvent être constituées de l'extrait de parfum lui-même ; dans ce cas, le parfum comprend des corps gras. Les huiles de la phase grasse peuvent être volatiles ou non et sont choisies en fonction de leurs paramètres de solubilité et de leur structure chimique. L'huile utilisée ou le mélange d'huiles utilisées présente de préférence des paramètres moyens de solubilité de Hansen dD, dP et dH à 25 °C qui satisfont aux trois conditions suivantes :

$$(1)\ dD \leq 20\ (J/cm^3)^{\frac{1}{2}}$$

$$(2)\ dP \leq 10\ (J/cm^3)^{\frac{1}{2}}$$

$$(3)\ dH \leq 15\ (J/cm^3)^{\frac{1}{2}}.$$

La définition des solvants dans l'espace de solubilité tridimensionnel selon Hansen est décrite dans l'article de C. M. Hansen : "The three dimensionnal solubility parameters" J. Paint Technol. 39, 105 (1967). Cet espace est défini par les paramètres dD, dP, dH ; ils sont exprimés en $(J/cm^3)^{\frac{1}{2}}$:

- dD caractérise les forces de dispersion de London issues de la formation de dipôles induits lors des chocs moléculaires ;

- dP caractérise les forces d'interactions de Debye entre dipôles permanents ainsi que les forces d'interactions de Keesom entre dipôles induits et dipôles permanents ;
- dH caractérise les forces d'interactions spécifiques (type liaisons hydrogène, acide/base, donneur/accepteur, etc...).

Les huiles hydrocarbonées peuvent être choisies parmi les huiles d'origine animale, les huiles d'origine végétale, les huiles de synthèse telles que l'isoparaffine hydrogénée, les esters et les éthers de synthèse et leurs mélanges.

Les huiles de silicone peuvent être choisies parmi les polysiloxanes linéaires liquides ou pâteux à température ambiante tels que les alkylpolysiloxanes, les alkylphénylpolysiloxanes, les alkylpolydiméthylsiloxanes et les polysiloxanes cycliques comme l'octaméthylcyclopentasiloxane, le décaméthylcyclopentasiloxane, ou leurs mélanges.

Les huiles de silicone peuvent être choisies aussi parmi les huiles appropriées comme co-solvants de l'organopolysiloxane élastomérique et du parfum.

Ces huiles appropriées comme co-solvants sont par exemple les silicones volatiles comportant une structure silicone linéaire et des motifs à chaîne alkyle pendante et/ou en bout de structure silicone, ces chaînes alkyle étant linéaire ou ramifiées et comportant 3 à 10 atomes de carbone. Les silicones volatiles à chaîne alkyle présentent notamment la formule (1) suivante :

$$R_1(CH_3)_2Si\text{-}O\text{-}\{\text{-}Si\text{-}(CH_3)R_2\text{-}O\text{-}\}_n\text{-}\{\text{-}Si\text{-}(CH_3)_2\text{-}O\text{-}\}_m\text{-}Si(CH_3)_2R_1$$

où $R_1$ et $R_2$ sont indépendamment H, méthyle ou une chaîne alkyle linéaire ou ramifiée ayant de 3 à 10 atomes de carbone, n et m étant des entiers allant de 0 à 10, à condition que si $R_1$ vaut H ou méthyle, n soit différent de 0 et $R_2$ représente une chaîne alkyle de 3 à 10 atomes.

Avantageusement les silicones volatiles de formule (1) ont une masse moléculaire allant de 290 à 3 000 et une volatilité ou taux d'évaporation Nv généralement compris entre 150 et 500 secondes (temps correspondant à l'évaporation de 0,2 ml de silicone volatile à 23°C dans une atmosphère stable à 50 % d'humidité relative).

Comme silicones volatiles à chaîne alkyle utilisables dans l'invention, on peut citer les alkylheptaméthyltrisiloxanes avec un groupe alkyle comportant de 4 à 8 atomes de carbone comme par exemple l'hexylheptaméthyltrisiloxane de formule : $(CH_3)_3\text{-}Si\text{-}O\text{-}Si(CH_3)(C_6H_{13})\text{-}O\text{-}Si(CH_3)_3$ ; l'octylheptaméthyltrisiloxane de formule : $(CH_3)_3\text{-}Si\text{-}O\text{-}Si(CH_3)(C_8H_{15})\text{-}O\text{-}Si(CH_3)_3$; et leurs mélanges.

De préférence, l'huile utilisée comme co-solvant est l'hexylheptaméthyltrisiloxane.

Le co-solvant est en particulier présent pour obtenir une composition sous forme de gel.

Le parfum peut comprendre tout parfum et mélange de parfum d'origine naturelle ou synthétique, y compris les huiles essentielles. Le parfum peut être présent dans la composition en une quantité allant de 0,001 à 50 % et de préférence de 0,1 à 30 % du poids total de la composition. La phase grasse est présente en une quantité allant de 0,1 à 80 %.

De façon préférentielle, le ou les organopolysiloxanes élastomériques utilisés selon l'invention sont présents, à une concentration en matière active, allant de 0,1 à 50 % du poids total de la composition. Toutefois, ces proportions d'organopolysiloxane ainsi que celles de la phase grasse peuvent varier suivant la forme galénique que l'on souhaite obtenir.

La composition selon l'invention se présente de préférence sous forme de gel et comprend, par rapport au poids total de la composition. 0,001 à 50 % et mieux 0,01 à 50 % de parfum, 1 à 50 % et mieux 20 à 50 % d'organopolysiloxane et 10 à 80 % et mieux 20 à 70 % de phase grasse et au moins une huile appropriée comme co-solvant du parfum et de l'organopolysiloxane élastomérique, telle que définie ci-dessus.

La composition selon l'invention peut être appropriée notamment pour une utilisation topique et constituer en particulier une composition cosmétique et/ou dermatologique. Elle contient alors un milieu physiologiquement acceptable. On entend par "physiologiquement acceptable" un milieu compatible avec la peau, les yeux et les fibres kératiniques des êtres humains.

Comme indiquée ci-dessus, la composition de l'invention se présente de préférence sous forme d'un gel. De façon connue, la composition de l'invention peut contenir également les adjuvants habituels dans les domaines cosmétique et dermatologique, tels que les corps gras, les actifs hydrophiles ou lipophiles, les conservateurs, les filtres, les pigments (par exemple nacres) et les matières colorantes. Les quantités de ces différents adjuvants sont celles classiquement utilisées dans les domaines considérés, et par exemple de 0,01 à 20 % du poids total de la composition. Bien entendu, ces adjuvants doivent être de nature et utilisés en quantité telle qu'ils ne perturbent pas la composition de l'invention.

Comme corps gras utilisables dans l'invention, on peut citer en plus des huiles indiquées ci-dessus les cires, notamment les cires hydrocarbonées ou de silicone.

La composition peut être utilisée notamment comme produit de nettoyage, de soin et/ou de parfumage de la peau et/ou des cheveux, et/ou comme produit de maquillage tout en assurant le parfumage de la peau et/ou des cheveux. La composition peut aussi être utilisée uniquement comme produit de parfumage.

Aussi, la présente invention se rapporte également à un procédé cosmétique de parfumage et/ou de nettoyage de la peau et/ou des cheveux consistant à appliquer sur la peau et/ou les cheveux une composition comprenant, dans un milieu physiologiquement acceptable, au moins un organopolysiloxane élastomérique solide au moins partiellement réticulé associé à une phase grasse, contenant au moins un parfum.

Les exemples de compositions données ci-après sont donnés à titre illustratif et non limitatif. Les quantités sont données en pourcentage en poids.

### Exemple 1 : Gel parfumé

- Organopolysiloxane réticulé, à 60 % dans PDMS non volatil (KSG6)      35 %
- Parfum      10 %
- Hexylheptaméthyl trisiloxane (DC2-1731 Votatile Fluid vendu par Dow Corning)      55 %

On obtient un gel parfumé doux à l'application, ayant une forte rémanence dans le temps.

### Revendications

1. Utilisation d'au moins un organopolysiloxane solide élastomérique au moins partiellement réticulé associé à une phase grasse dans une composition cosmétique contenant, dans un milieu physiologiquement acceptable, un parfum pour la fixation et/ou la libération prolongée de ce parfum, l'organo-polysiloxane élastomérique étant susceptible d'être obtenu par réaction d'addition et de réticulation, en présence d'un catalyseur, d'au moins :

   (a) un organopolysiloxane ayant au moins deux groupes alcényle inférieurs par molécule ; et
   (b) un organopolysiloxane ayant au moins deux atomes d'hydrogène liés à un atome de silicium par molécule.

2. Utilisation selon la revendication 1, caractérisée en ce que l'organopolysiloxane est choisi parmi :

   - i) les organopolysiloxanes comprenant des motifs $R_2SiO$ et $RSiO_{1,5}$ et éventuellement des motifs $R_3SiO_{0,5}$ et/ou $SiO_2$, dans lesquels les radicaux R, indépendamment les uns des autres, représentent un hydrogène, un alkyle, un aryle, un groupe aliphatique insaturé, le rapport en poids des motifs $R_2SiO$ sur les motifs $RSiO_{1,5}$ allant de 1/1 à 30/1 ;
   - ii) les organopolysiloxanes insolubles dans l'huile de silicone, susceptibles d'être obtenus par addition d'un organohydrogénopolysiloxane (1) et d'un organopolysiloxane (2) ayant des groupes aliphatiques insaturés de telle sorte que la quantité d'hydrogène ou de groupes aliphatiques insaturés dans respectivement (1) et (2) soit comprise entre 1 et 20 mole % lorsque l'organopolysiloxane est non-cyclique et entre 1 et 50 mole % lorsque l'organopolysiloxane est cyclique.

3. Utilisation selon l'une quelconque des revendications précédentes, caractérisée en ce que l'organopolysiloxane solide élastomérique est présent, en matière active, en une concentration allant de 0,1 à 50 % du poids total de la composition.

4. Utilisation selon l'une quelconque des revendications précédentes, caractérisée en ce que la phase grasse comprend au moins une huile choisie parmi les huiles hydrocarbonées, les huiles siliconées, les huiles fluorées et leurs mélanges.

5. Utilisation selon la revendication précédente, caractérisée en ce que l'huile hydrocarbonée est choisie parmi les huiles d'origine animale, les huiles d'origine végétale, les huiles de synthèse et leurs mélanges.

6. Utilisation selon la revendication 4, caractérisée en ce que l'huile siliconée est choisie parmi les polysiloxanes linéaires liquides ou pâteux à température ambiante, les polysiloxanes cycliques et leurs mélanges.

7. Utilisation selon l'une quelconque des revendications précédentes, caractérisée en ce que la phase grasse contient au moins une huile appropriée comme co-solvant du parfum et de l'organopolysiloxane élastomérique.

8. Utilisation selon la revendication précédente, caractérisée en ce que le co-solvant présente la formule suivante :

$$R_1(CH_3)_2Si\text{-}O\text{-}\{\text{-}Si\text{-}(CH_3)R_2\text{-}O\text{-}\}_n\text{-}\{\text{-}Si\text{-}(CH_3)_2\text{-}O\text{-}\}_m\text{-}Si(CH_3)_2R_1$$

où $R_1$ et $R_2$ sont indépendamment H, méthyle ou une chaîne alkyle linéaire ou ramifiée ayant de 3 à 10 atomes de carbone, n et m étant des entiers allant de 0 à 10, à condition que si $R_1$ vaut H ou méthyle, n soit différent de 0 et $R_2$ représente une chaîne alkyle de 3 à 10 atomes.

9. Utilisation selon la revendication 7 ou 8, caractérisée en ce que le co-solvant est choisi parmi l'hexylheptaméthyltrisiloxane, l'octylheptaméthyltrisiloxane et leurs mélanges.

10. Utilisation selon l'une quelconque des revendications précédentes, caractérisée en ce que le parfum est présent en une quantité allant de 0,001 à 50 % du poids total de la composition.

11. Utilisation selon l'une quelconque des revendications précédentes, caractérisée en ce que la phase grasse est présente en une quantité allant de 0,1 à 80 %.

12. Utilisation selon l'une quelconque des revendications précédentes, caractérisée en ce que la composition se présente sous forme de gel.

13. Utilisation selon la revendication précédente, caractérisée en ce que la composition comprend, par rapport au poids total de la composition, 0,001 à 50 % de parfum, 1 à 50 % d'organopolysiloxane et 10 à 80 % de phase grasse.

14. Utilisation selon la revendication précédente, caractérisée en ce que la composition comprend, par rapport au poids total de la composition, 0,01 à 50 % de parfum, 20 à 50 % d'organopolysiloxane, 20 à 70 % de phase grasse.

15. Utilisation selon l'une quelconque des revendications précédentes, caractérisée en ce que la composition comprend en outre au moins un ingrédient choisi parmi les corps gras, les actifs, les filtres, les pigments, les matières colorantes et leurs mélanges.

16. Utilisation selon l'une des revendications précédentes, caractérisée en ce que la composition est une composition de nettoyage et/ou de soin de la peau.

17. Utilisation selon l'une des revendications précédentes, caractérisée en ce que la composition est une composition de parfumage de la peau.

18. Procédé cosmétique de parfumage et/ou de nettoyage de la peau et/ou des cheveux consistant à appliquer sur la peau et/ou les cheveux une composition comprenant, dans un milieu physiologiquement acceptable, au moins un organopolysiloxane élastomérique solide au moins partiellement réticulé associé à une phase grasse, contenant au moins un parfum, l'organo-polysiloxane élastomérique étant susceptible d'être obtenu par réaction d'addition et de réticulation, en présence d'un catalyseur, d'au moins :

(a) un organopolysiloxane ayant au moins deux groupes alcényle inférieurs par molécule ; et
(b) un organopolysiloxane ayant au moins deux atomes d'hydrogène liés à un atome de silicium par molécule.

19. Composition de parfumage contenant un parfum, caractérisée en ce qu'elle comprend en outre par rapport au poids total de la composition, 0,001 à 50 % de parfum, 1 à 50 % d'organopolysiloxane et 10 à 80 % de phase grasse et au moins une huile appropriée comme co-solvant du parfum et de l'organopolysiloxane élastomérique, présentant la formule suivante :

$$R_1(CH_3)_2Si\text{-}O\text{-}\{\text{-}Si\text{-}(CH_3)R_2\text{-}O\text{-}\}_n\text{-}\{\text{-}Si\text{-}(CH_3)_2\text{-}O\text{-}\}_m\text{-}Si(CH_3)_2R_1$$

où $R_1$ et $R_2$ sont indépendamment H, méthyle ou une chaîne alkyle linéaire ou ramifiée ayant de 3 à 10 atomes de carbone, n et m étant des entiers allant de 0 à 10, à condition que si $R_1$ vaut H ou méthyle, n soit différent de

EP 0 857 481 B1

0 et $R_2$ représente une chaîne alkyle de 3 à 10 atomes.

**Patentansprüche**

1. Verwendung mindestens eines festen, elastomeren, mindestens zum Teil vernetzten Organopolysiloxans in Kombination mit einer Fettphase in einer kosmetischen Zusammensetzung, die in einem physiologisch akzeptablen Medium ein Parfum enthält, zur Fixierung und/oder protrahierten Freisetzung des Parfums, wobei das elastomere Organopolysiloxan durch Addition und Vernetzung von mindestens:

   - (a) einem Organopolysiloxan, das mindestens zwei niedere Alkenylgruppen pro Molekül aufweist; und
   - (b) einem Organopolysiloxan, das mindestens zwei Wasserstoffatome, die an ein Siliciumatom gebunden sind, pro Molekül aufweist,

   in Gegenwart eines Katalysators hergestellt werden kann.

2. Verwendung nach Anspruch 1, dadurch gekennzeichnet, dass das Organopolysiloxan ausgewählt ist unter:

   - i) den Organopolysiloxanen, die $R_2SiO$- und $RSiO_{1,5}$-Einheiten und gegebenenfalls $R_3SiO_{0,5}$- und/oder $SiO_2$-Einheiten aufweisen, worin die Gruppen R unabhängig voneinander Wasserstoff, eine Alkylgruppe, eine Arylgruppe oder eine ungesättigte aliphatische Gruppe bedeuten, wobei das Gewichtsverhältnis von $R_2SiO$-Einheiten zu $RSiO_{1,5}$-Einheiten im Bereich von 1/1 bis 30/1 liegt;
   - ii) den in Siliconöl unlöslichen Organopolysiloxanen, die durch Addition eines Organohydrogenopolysiloxans (1) und eines Organopolysiloxans mit ungesättigten aliphatischen Gruppen (2) hergestellt werden, wobei sie so gewählt werden, dass der Mengenanteil von Wasserstoff oder ungesättigten aliphatischen Gruppen in (1) bzw. (2) im Bereich von 1 bis 20 Mol-%, wenn das Organopolysiloxan nicht cyclisch ist, und im Bereich von 1 bis 50 Mol-% liegt, wenn das Organopolysiloxan cyclisch vorliegt.

3. Verwendung nach einem der vorhergehenden Ansprüche, dadurch gekennzeichnet, dass das feste elastomere Organopolysiloxan in einer Wirkstoffkonzentration von 0,1 bis 50 % des Gesamtgewichts der Zusammensetzung vorliegt.

4. Verwendung nach einem der vorhergehenden Ansprüche, dadurch gekennzeichnet, dass die Fettphase mindestes ein Öl enthält, das unter den Kohlenwasserstoffölen, Siliconölen, fluorierten Ölen und deren Gemischen ausgewählt ist.

5. Verwendung nach dem vorhergehenden Anspruch, dadurch gekennzeichnet, dass das Kohlenwasserstofföl unter den Ölen tierischen Ursprungs, Ölen pflanzlichen Ursprungs, synthetischen Ölen und deren Gemischen ausgewählt ist.

6. Verwendung nach Anspruch 4, dadurch gekennzeichnet, dass das Siliconöl unter den bei Raumtemperatur flüssigen oder pastösen, geradkettigen Polysiloxanen oder den cyclischen Polysiloxanen und deren Gemischen ausgewählt ist.

7. Verwendung nach einem der vorhergehenden Ansprüche, dadurch gekennzeichnet, dass die Fettphase mindestens ein Öl enthält, das als Colösungsmittel des Parfums und des elastomeren Organopolysiloxans geeignet ist.

8. Verwendung nach dem vorhergehenden Anspruch, dadurch gekennzeichnet, dass das Colösungsmittel die folgende Formel aufweist:

$$R_1(CH_3)_2Si\text{-}O\text{-}\{\text{-}Si(CH_3)R_2\text{-}O\text{-}\}_n\text{-}\{\text{-}Si\text{-}(CH_3)_2\text{-}O\text{-}\}_m\text{-}Si(CH_3)_2R_1,$$

worin $R_1$ und $R_2$ unabhängig voneinander H, Methyl oder eine geradkettige oder verzweigte Alkylgruppe mit 3 bis 10 Kohlenstoffatomen bedeuten, wobei n und m 0 oder eine ganze Zahl im Bereich von 1 bis 10 bedeuten, mit der Maßgabe, dass n von 0 verschieden ist und $R_2$ eine Alkylgruppe mit 3 bis 10 Kohlenstoffatomen bedeutet, wenn $R_1$ Wasserstoff oder Methyl bedeutet.

7

**9.** Verwendung nach Anspruch 7 oder 8, dadurch gekennzeichnet, dass das Colösungsmittel unter Hexylheptamethyltrisiloxan, Octylheptamethyltrisiloxan und deren Gemischen ausgewählt ist.

**10.** Verwendung nach einem der vorhergehenden Ansprüche, dadurch gekennzeichnet, dass das Parfum in einer Menge von 0,001 bis 50 % des Gesamtgewichts der Zusammensetzung vorliegt.

**11.** Verwendung nach einem der vorhergehenden Ansprüche, dadurch gekennzeichnet, dass die Fettphase in einer Menge von 0,1 bis 80 % vorliegt.

**12.** Verwendung nach einem der vorhergehenden Ansprüche, dadurch gekennzeichnet, dass die Zusammensetzung in Gelform vorliegt.

**13.** Verwendung nach dem vorhergehenden Anspruch, dadurch gekennzeichnet, dass die Zusammensetzung, bezogen auf das Gesamtgewicht der Zusammensetzung, 0,001 bis 50 % Parfum, 1 bis 50 % Organopolysiloxan und 10 bis 80 % Fettphase enthält.

**14.** Verwendung nach dem vorhergehenden Anspruch, dadurch gekennzeichnet, dass die Zusammensetzung, bezogen auf das Gesamtgewicht der Zusammensetzung, 0,01 bis 50 % Parfum, 20 bis 50 % Organopolysiloxan und 20 bis 70 % Fettphase enthalt.

**15.** Verwendung nach einem der vorhergehenden Ansprüche, dadurch gekennzeichnet, dass die Zusammensetzung ferner mindestens einen Bestandteil enthält, der unter Fettsubstanzen, Wirkstoffen, Filtern, Pigmenten und Färbemittel und deren Gemischen ausgewählt ist.

**16.** Verwendung nach einem der vorhergehenden Ansprüche, dadurch gekennzeichnet, dass die Zusammensetzung eine Zusammensetzung für die Reinigung und/oder Pflege der Haut darstellt.

**17.** Verwendung nach einem der vorhergehenden Ansprüche, dadurch gekennzeichnet, dass die Zusammensetzung eine Zusammensetzung für die Parfümierung der Haut ist.

**18.** Kosmetisches Verfahren zur Parfümierung und/oder Reinigung der Haut und/oder der Haare, das darin besteht, auf die Haut und/oder die Haare eine Zusammensetzung aufzutragen, die in einem physiologisch akzeptablen Medium mindestens ein zumindest teilweise vemetztes, festes, elastomeres Organopolysiloxan in Kombination mit einer Fettphase enthält, die mindestes ein Parfum enthält, wobei das elastomere Organopolysiloxan in Gegenwart eines Katalysators durch Addition und Vernetzung von mindestens:

- (a) einem Organopolysiloxan, das mindestens zwei niedere Alkenylgruppen pro Molekül aufweist; und
- (b) einem Organopolysiloxan, das mindestens zwei Wasserstoffatome, die an ein Siliciumatom gebunden sind, pro Molekül aufweist,

hergestellt werden kann.

**19.** Zusammensetzung zur Parfümierung, die ein Parfum enthält, dadurch gekennzeichnet, dass sie ferner, bezogen auf das Gesamtgewicht der Zusammensetzung, 0,001 bis 50 % Parfum, 1 bis 50 % Organopolysiloxan und 10 bis 80 % Fettphase und mindestens ein als Colösungsmittel des Parfums und elastomeren Polyorganosiloxans geeignetes Öl enthalt, das die folgende Formel aufweist:

$$R_1(CH_3)_2Si\text{-}O\text{-}\{\text{-}Si(CH_3)R_2\text{-}O\text{-}\}_n\text{-}\{\text{-}Si\text{-}(CH_3)_2\text{-}O\text{-}\}_m\text{-}Si(CH_3)_2R_1,$$

worin $R_1$ und $R_2$ unabhängig voneinander H, Methyl oder eine geradkettige oder verzweigte Alkylgruppe mit 3 bis 10 Kohlenstoffatomen bedeuten, wobei n und m 0 oder eine ganze Zahl im Bereich von 1 bis 10 bedeuten, mit der Maßgabe, dass n von 0 verschieden ist und $R_2$ eine Alkylgruppe mit 3 bis 10 Kohlenstoffatomen bedeutet, wenn $R_1$ Wasserstoff oder Methyl bedeutet.

**Claims**

1. Use of at least one at least partially crosslinked, solid, elastomeric organopolysiloxane associated with a fatty phase, in a cosmetic composition containing, in a physiologically acceptable medium, a perfume for the fixing and/ or sustained release of this perfume, it being possible for the elastomeric organopolysiloxane to be obtained by an addition and crosslinking reaction, in the presence of a catalyst, of at least:

    (a) one organopolysiloxane having at least two lower alkenyl groups per molecule;
    (b) one organopolysiloxane having at least two hydrogen atoms linked to a silicon atom per molecule.

2. Use according to Claim 1, characterized in that the organopolysiloxane is chosen from:

    - i) organopolysiloxanes comprising units $R_2SiO$ and $RSiO_{1.5}$ and optionally units $R_3SiO_{0.5}$ and/or $SiO_2$ in which the radicals R, independently of each other, denote a hydrogen, an alkyl, an aryl, an unsaturated aliphatic group and in which the weight ratio of the units $R_2SiO$ to the units $RSiO_{1.5}$ ranges from 1/1 to 30/1;
    - ii) organopolysiloxanes which are insoluble in silicone oil, can be obtained by addition of an organohydrogenopolysiloxane (1) and of an organopolysiloxane (2) having unsaturated aliphatic groups, such that the amount of hydrogen or of unsaturated aliphatic groups in (1) and (2) respectively is between 1 and 20 mol% when the organopolysiloxane is non-cyclic and between 1 and 50 mol% when the organopolysiloxane is cyclic.

3. Use according to any one of the preceding claims, characterized in that the solid elastomeric organopolysiloxane is present, as active material, at a concentration ranging from 0.1 to 50% of the total weight of the composition.

4. Use according to any one of the preceding claims, characterized in that the fatty phase comprises at least one oil chosen from hydrocarbon oils, silicone oils and fluoro oils, and mixtures thereof.

5. Use according to the preceding claim, characterized in that the hydrocarbon oil is chosen from oils of animal origin, oils of plant origin and synthetic oils, and mixtures thereof.

6. Use according to Claim 4, characterized in that the silicone oil is chosen from linear polysiloxanes which are liquid or pasty at room temperature and cyclic polysiloxanes, and mixtures thereof.

7. Use according to any one of the preceding claims, characterized in that the fatty phase contains at least one oil which is suitable as a co-solvent for the perfume and for the elastomeric organopolysiloxane.

8. Use according to the preceding claim, characterized in that the co-solvent has the following formula:

$$R_1(CH_3)_2Si\text{-}O\text{-}\{\text{-Si-}(CH_3)R_2\text{-O-}\}_n\text{-}(\text{-Si-}(CH_3)_2\text{-O-})_m\text{-Si}(CH_3)_2R_1$$

where $R_1$ and $R_2$ are, independently, H, methyl or a linear or branched alkyl chain having from 3 to 10 carbon atoms, n and m being integers ranging from 0 to 10, on condition that if $R_1$ is H or methyl, n is other than 0 and $R_2$ represents an alkyl chain of 3 to 10 atoms.

9. Use according to Claim 7 or 8, characterized in that the co-solvent is chosen from hexylheptamethyltrisiloxane and octylheptamethyltrisiloxane, and mixtures thereof.

10. Use according to any one of the preceding claims, characterized in that the perfume is present in an amount ranging from 0.001 to 50% of the total weight of the composition.

11. Use according to any one of the preceding claims, characterized in that the fatty phase is present in an amount ranging from 0.1 to 80%.

12. Use according to any one of the preceding claims, characterized in that the composition is in gel form.

13. Use according to the preceding claim, characterized in that the composition comprises, relative to the total weight of the composition, 0.001 to 50% of perfume, 1 to 50% of organopolysiloxane and 10 to 80% of fatty phase.

**14.** Use according to the preceding claim, characterized in that the composition comprises, relative to the total weight of the composition, 0.01 to 50% of perfume, 20 to 50% of organopolysiloxane and 20 to 70% of fatty phase.

**15.** Use according to any one of the preceding claims, characterized in that the composition also comprises at least one ingredient chosen from fatty substances, active agents, screening agents, pigments and dyestuffs, and mixtures thereof.

**16.** Use according to one of the preceding claims, characterized in that the composition is a composition to cleanse and/or care for the skin.

**17.** Use according to one of the preceding claims, characterized in that the composition is a composition for perfuming the skin.

**18.** Cosmetic process to perfume and/or cleanse the skin and/or the hair, this process consisting in applying to the skin and/or the hair a composition comprising, in a physiologically acceptable medium, at least one at least partially crosslinked, solid, elastomeric organopolysiloxane associated with a fatty phase, containing at least one perfume, it being possible for the elastomeric organopolysiloxane to be obtained by an addition and crosslinking reaction, in the presence of a catalyst, of at least:

(a) one organopolysiloxane having at least two lower alkenyl groups per molecule; and
(b) one organopolysiloxane having at least two hydrogen atoms linked to a silicon atom per molecule.

**19.** Perfume composition containing a perfume, characterized in that it also comprises, relative to the total weight of the composition, 0.001 to 50% of perfume, 1 to 50% of organopolysiloxane and 10 to 80% of fatty phase and at least one oil which is suitable as a co-solvent for the perfume and for the elastomeric organopolysiloxane, having the following formula:

$$R_1(CH_3)_2Si\text{-}O\text{-}\{\text{-}Si\text{-}(CH_3)R_2\text{-}O\text{-}\}_n\text{-}(\text{-}Si\text{-}(CH_3)_2\text{-}O\text{-}\}_m\text{-}Si(CH_3)_2R_1$$

where $R_1$ and $R_2$ are, independently, H, methyl or a linear or branched alkyl chain having from 3 to 10 carbon atoms, n and m being integers ranging from 0 to 10, on condition that if $R_1$ is H or methyl, n is other than 0 and $R_2$ represents an alkyl chain of 3 to 10 atoms.